# EUROPEAN PATENT APPLICATION

(11) **EP 3 903 768 A1**
(43) Date of publication of application: **03.11.2021**
(21) Application number: 21170914.2
(22) Date of filing: 28.04.2021
(51) Int. Cl.: A61K 9/08, A61K 47/10, A61K 47/22, A61K 47/44, A61K 31/385, A61P 3/00

(54) **INJECTABLE FORMULATIONS OF LIPOIC ACID FOR THE TREATMENT OF OXIDATIVE STRESS AND METABOLIC ALTERATIONS**

(30) Priority: 30.04.2020 IT 202000009577
(71) Applicant: FATRO S.p.A., 40064 Ozzano dell'Emilia (BO) (IT)
(72) Inventor: CORACE, Giuseppe, 40064 Ozzano dell'Emilia (BO) (IT); FARNE', Eleonora, 40064 Ozzano dell'Emilia (BO) (IT); MONTECCHI, Lauretta, 40064 Ozzano dell'Emilia (BO) (IT); MORBIDELLI, Eva, 40064 Ozzano dell'Emilia (BO) (IT)
(74) Representative: Bianchetti & Minoja SRL

(57) **Abstract**

The present invention relates to non-aqueous injectable formulations of lipoic acid or enantiomers thereof which are useful for the prevention or treatment of pathologies caused by oxidative stress and metabolic disorders, and to a method for their stabilisation.

## Description

The present invention relates to non-aqueous injectable formulations of lipoic acid or the enantiomers thereof which are useful for the prevention or treatment of pathologies caused by oxidative stress and metabolic disorders. The invention also relates to a method for the stabilisation of said formulations.

### Prior art

Lipoic acid or thioctic acid (1,2-dithiolan-3-pentanoic acid) is a substance known for its antioxidant and metabolic properties, which is widely used in human and veterinary clinical practice.

Lipoic acid (ALA) has a chiral centre, involving the presence of two isomers: R-ALA and S-ALA. The R-ALA isomer is present in nature, and is the more active form. The racemate, produced by synthesis, is commonly used in treatment.

Injectable formulations containing ALA as active ingredient exist on the market. They include Thioctacid 600 T® (Meda Pharma), containing 2.5% ALA in aqueous solution as trometamol salt, which is indicated for human use in the treatment of diabetic polyneuropathy; Erbacolina plus® (Ceva) injectable solution for veterinary use containing 0.025% ALA, used as liver protector; and Metabolase® (Fatro) injectable solution for veterinary use containing 0.02% ALA, used as a fatigue-relieving and detoxifying agent and for various kinds of oxidative stress. The concentration of ALA in the above-mentioned products never exceeds 2.5% due to its chemical instability in aqueous solution, which increases with the ALA concentration. The oral administration route presents problems associated with the low bioavailability of ALA. In fact, ALA is absorbed rapidly and almost completely when administered orally, but has low bioavailability (about 30%) due to high pre-systemic metabolisation. Its bioavailability further declines in the presence of food.

Although numerous studies have been conducted with a view to increasing the oral bioavailability of ALA, the parenteral route remains the preferred route, especially in the veterinary field, in particular for livestock. To avoid injecting high volumes of solution, the formulations must contain high concentrations of ALA.

Parenteral formulations allow greater precision of the dose administered according to the live weight of the animal, and are the pharmaceutical forms most widely used in the livestock field for the treatment of debilitating disorders.

ALA exists in both oxidised form (LA, lipoic acid) and reduced form (DHLA, dihydrolipoic acid).

In solution, ALA can be present as a balance between the two forms, LA and DHLA, depending on the physicochemical conditions of the solution.

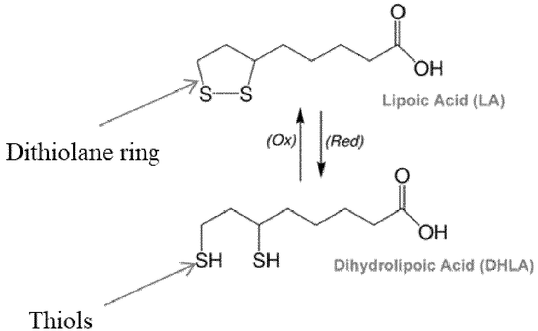

It has been demonstrated that the reduced form DHLA aids polymerisation with the formation of intermolecular disulphide bonds (Kisanuki A - Polymer Chemistry 2010, 48, 5247-53). Thus maintaining ALA in oxidised form involves lower degradation of ALA and consequently greater stability and therapeutic efficacy.

Various formulations designed to stabilise or increase the bioavailability of ALA have been described.

EP2736507 discloses aqueous solutions for oral use with propylene glycol at various pH values ranging from 7.0-9.5. The aqueous solution with 30% propylene glycol containing 60 mg/ml of ALA proved to be the most stable, but with a decline from 95.6 to 90.3 at 25°C in 9 months. The advantage of this oral solution was greater solubility and stability in the gastric juices than solid comparator formulations.

US7030154 discloses a method of stabilising the R-ALA isomer with nicotinamide. Formulations in the form of pastilles, capsules and solution were used to treat oxidative stress.

EP0318891 discloses aqueous injectable solutions containing trometamol salts and amino acids with lipoic acid. The maximum concentration of lipoic acid is 50 mg/ml, but the stability data of the solutions are not described.

US5728735A discloses oral and injectable compositions of R or S lipoic acid for anti-inflammatory use. The injectable formulation described is a salt with trometamol.

CN102525930 discloses injectable formulations of lipoic acid in liposomes with increased stability and bioavailability.

CN107789317 describes aqueous injectable solutions of lipoic acid with hydroxypropyl-cyclodextrin having increased stability.

US 7 772 274 discloses non-aqueous formulations of docetaxel, also containing lipoic acid as stabilising agent.

The stability of ALA in solution declines considerably when the ALA concentration in the solution increases. In fact, according to EP2736507, cited above, the stability of an ALA solution at a concentration of 120 mg/ml falls by about 30% in 7 days, whereas at a concentration of 60 mg/ml, conditions being equal, the stability falls by about 2%.

It would therefore be desirable to have injectable formulations containing a high concentration of ALA, possessing high stability and high bioavailability.

### Description of the invention

It has now surprisingly been found that non-aqueous solutions of ALA, containing non-ionic surfactants dissolved in polar organic solvents, are particularly stable. Said stability has also been observed at high ALA concentrations.

The object of the invention is therefore non-aqueous injectable formulations comprising ALA or the isomers thereof as active ingredient at a concentration ranging from 2.5% to 25% w/v, at least one non-ionic surfactant at a concentration ranging from 2.5% to 15% w/v, and a polar solvent at a concentration ranging from 50% to 95% w/v. The compositions according to the invention preferably contain lipoic acid as the sole active ingredient or combined with L-carnitine.

Examples of non-ionic surfactants include polysorbates, polyethoxylated fatty acids, polyethoxylated fatty alcohols and polyethoxylated tocopherols, in particular tocopheryl polyethylene glycol succinates (TPGS).

TPGS are non-ionic surfactants used in the pharmaceutical field for their properties of emulsifying and solubilising poorly soluble products. TPGS or vitamin E polyethylene glycol succinate is a substance obtained by esterification of d-alpha-tocopherol acid succinate with polyethylene glycol. Depending on the polyethylene glycol chain, various types of TPGS, from 200 to 6000, can be produced.

As well as being a non-ionic surfactant, TPGS is a highly bioavailable form of vitamin E, and can therefore perform an antioxidant action additional to that of ALA. TPGS has been used to enhance the bioavailability of poorly soluble products, such as coenzyme Q10 (WO 2006138431). Stable injectable aqueous solutions of docetaxel have been obtained by using TPGS as solubiliser and ALA and other excipients as antioxidant (US8912228).

The TPGS preferably used in the formulations according to the invention is TPGS 1000.

The polyethylene glycol chain of TPGS 1000 (cas. 9002-96-4) has a molecular weight of 1000 g/mol. It is the most widely used form of TPGS in the pharmaceutical field, is approved by the FDA as a diet supplement, and a monograph thereof is present in the US Pharmacopoeia.

Examples of polar organic solvents include alcohols, glycols and derivatives thereof (glycerol formal), lactams, and all polar organic solvents usable in the pharmaceutical field in general.

The formulation according to the invention preferably consists of ALA at a concentration ranging from 5% to 15%, a non-ionic surfactant ranging from 5% to 15% and a polar solvent ranging from 70% to 85% (all percentages expressed as weight/volume).

The non-ionic surfactant is preferably TPGS at a concentration ranging between 2.5% and 15% w/v, and more preferably between 5% and 15% w/v. Such a concentration is crucial to the dissolution and stability of ALA at high concentrations.

The solvent is preferably glycerol formal or a mixture of glycerol formal and propylene glycol in a weight ratio ranging from 6:4 to 8:2.

The formulations according to the invention can also contain amino-acid derivatives, such as carnitine in the form of a base, salt or acylated derivatives, for example L-carnitine, acetyl-L-carnitine, arginine and lysine.

Various other formulations with sundry active ingredients (anti-inflammatories, antitumorals or antibiotics) have been described, containing TPGS as solubiliser and small amounts of ALA with antioxidant functions.

No formulations of stable injectable solutions with high concentrations of ALA or the isomers thereof are known for use in the disorders for which ALA is indicated, such as oxidative stress and metabolic disorders, like steatosis or necrosis of the liver.

The advantages of the formulations according to the invention are:
- Stability in solution of ALA or the isomers thereof which allows its storage under normal conditions for long periods (exceeding 24 months).
- High concentrations of ALA suitable for veterinary use, in particular for livestock.
- The possibility of storing the product at below-zero temperatures, without the risk of freezing or precipitation.
- As well as being a non-ionic surfactant, TPGS is a highly bioavailable form of vitamin E, and can therefore perform an antioxidant action additional to that of ALA.

A further advantage of the formulation according to the invention is that it avoids the precipitation of ALA in aqueous solutions. In fact, at the concentrations used, ALA would be insoluble in aqueous solutions and would tend to precipitate. Conversely, the claimed formulation allows:
- Extemporaneous dilution before administration of the anhydrous solution of ALA with aqueous solutions, without the risk of its precipitation.
- Maintenance of solubility in aqueous solutions allows the administration of ALA directly into the bloodstream (intravenous administration).

The invention is described in detail in the examples below.

### Example 1

10.0 g of TPGS 1000 is solubilised under stirring at 60°C, under nitrogen stream, in a mixture consisting of 70.0 g glycerol formal and 20 g propylene glycol. The resulting solution is cooled to about 25°C. 10 g of ALA is solubilised, and finally, the solution is made up to a volume of 100 ml with glycerol formal.

### Example 2

15.0 g of TPGS 1000 is solubilised under stirring at 60°C, under nitrogen stream, in a mixture consisting of 70.0 g glycerol formal and 20 g propylene glycol. The resulting solution is cooled to about 25°C. 15 g of ALA is solubilised, and finally, the solution is made up to a volume of 100 ml with glycerol formal.

### Example 3

7.5 g of TPGS 1000 is solubilised under stirring at 60°C, under nitrogen stream, in a mixture consisting of 80.0 g glycerol formal and 20 g propylene glycol. The resulting solution is cooled to about 25°C. 7.5 g of ALA is solubilised, and, the solution is made up to a volume of 100 ml with glycerol formal.

### Example 4

10.0 g of TPGS 1000 is solubilised under stirring at 60°C, under nitrogen stream, in a mixture consisting of 60.0 g glycerol formal and 16.4 g propylene glycol. The resulting solution is cooled to about 25°C. 10 g of ALA and 10.0 g of L-carnitine are solubilised, and finally, the solution is made up to a volume of 100 ml with glycerol formal.

### Example 5

10.0 g of TPGS 1000 is added to 92.7 g of glycerol formal under stirring at 60°C, under nitrogen stream. The resulting solution is cooled to about 25°C. 10 g of ALA is solubilised, and finally, the solution is made up to a volume of 100 ml with glycerol formal.

### Example 6

20.0 g of polyethoxylated castor oil (Kolliphor ELP) and 0.27 g of BHA are solubilised under stirring at 60°C, under nitrogen stream, in a mixture consisting of 60.0 g glycerol formal and 12.7 g 2-pyrrolidone. The resulting solution is cooled to about 25°C. 10 g of ALA and 10.0 g of L-carnitine are solubilised, and finally, the solution is made up to a volume of 100 ml with glycerol formal.

### Comparative Example 7

10 g of ALA is solubilised under nitrogen stream and under stirring in a mixture consisting of 65 g water, 30 g polyethylene glycol 300 and 9.5 g meglumine, and finally made up to a volume of 100 ml with water.

### Comparative Example 8

10 g of ALA is solubilised under nitrogen stream and under stirring in a mixture consisting of 65 g water, 30 g polyethylene glycol 300, 9.5 g meglumine and 1 g ascorbic acid, and finally made up to a volume of 100 ml with water.

### Comparative Example 9

10.0 g of TPGS 1000 is added to 85 g of water under stirring, at 60°C, under nitrogen stream. The resulting solution is cooled to about 25°C. 10 g of ALA is solubilised and finally, the solution is made up a volume of 100 ml with water.

### Example 10

20.0 g of TPGS 1000 is solubilised under stirring at 60°C, under nitrogen stream, in a mixture consisting of 60.0 g glycerol formal and 15 g propylene glycol. The resulting solution is cooled to about 25°C. 20 g of ALA is solubilised, and finally, the solution is made up to a volume of 100 ml with glycerol formal.

### Example 11

17.0 g of TPGS 1000 is solubilised under stirring at 60°C, under nitrogen stream, in a mixture consisting of 60.0 g glycerol formal and 15 g propylene glycol. The resulting solution is cooled to about 25°C. 17 g of ALA is solubilised, and finally, the solution is made up to a volume of 100 ml with glycerol formal.

### Example 12

2.5 g of TPGS 1000 is solubilised under stirring at 60°C, under nitrogen stream, in a mixture consisting of 80.0 g glycerol formal and 20 g propylene glycol. The resulting solution is cooled to about 25°C. 2.5 g of ALA is solubilised, and the solution is made up to a volume of 100 ml with glycerol formal.

### Example 13

A long-term stability study was conducted at 25°C, for a period of 24 months, on the ALA solutions prepared as in Examples 1-12. The results are shown in Table 1 below.

**Table 1 Stability of ALA solutions at 25°C, results expressed as a percentage of the assay value of ALA included in the formulation.**

| Example | To **(%)** | 10 days **(%)** | 1 month **(%)** | 3 months **(%)** | 6 months **(%)** | 12 months **(%)** | 18 months **(%)** | 24 months **(%)** |
|---|---|---|---|---|---|---|---|---|
| 1 | **101.32** | **101.12** | **101.01** | **101.21** | **100.91** | **100.85** | **101.06** | **100.76** |
| 2 | **100.36** | **100.31** | **100.11** | **99.89** | **100.15** | **100.03** | **99.79** | **99.95** |
| 3 | **102.36** | **102.28** | **102.21** | **102.09** | **101.95** | **102.22** | **101.88** | **101.74** |
| 4 | **103.26** | **103.21** | **103.03** | **102.88** | **102.47** | **102.36** | **102.03** | **101.85** |
| 5 | **100.25** | **100.03** | **100.14** | **99.87** | **99.96** | **100.10** | **100.19** | **99.91** |
| 6 | **101.26** | **100.87** | **100.59** | **99.58** | **98.16** | **97.78** | **97.56** | **97.17** |
| 7 | **100.5** | **74.33** | **68.36** | **46.22** | **40.74** | **32.86** | **20.21** | **15.26** |
| 8 | **101.11** | **69.06** | **64.32** | **32.03** | **19.77** | **10.32** | **7.39** | **4.26** |
| 9 | **100.36** | **82.36** | **75.29** | **68.22** | **60.18** | **56.29** | **47.58** | **39.14** |
| 10 | **100.24** | **100.08** | **100.01** | **99.89** | **97.54** | **95.66** | **93.21** | **92.11** |
| 11 | **101.28** | **100.87** | **100.61** | **99.27** | **98.05** | **96.46** | **94.78** | **93.55** |
| 12 | **100.68** | **100.52** | **100.49** | **100.31** | **100.29** | **100.19** | **100.15** | **100.08** |

As will be seen from Table 1, the anhydrous solutions (Examples 1-6 and 12) proved to be surprisingly stable, whereas the aqueous solutions (Comparative Examples 7-9) proved unstable.

TPGS 1000, performing the dual function of surfactant and antioxidant, stabilises ALA (Examples 1-5 and 12). In Example 6, the same stabilisation is obtained by adding the antioxidant BHA to the surfactant polyethoxylated castor oil. As will be seen from the same table, the concentration of TPGS 1000 plays a crucial role in the stabilisation of ALA. In fact, in Examples 10 and 11, wherein TPGS 1000 is included at concentrations of 20% and 17% respectively, it was observed that its stabilising activity towards ALA declines over time. After 18 months the assay value of ALA fell to below 95% in both examples, and this is incompatible with the pharmacopoeia specifications for ALA-based pharmaceutical products. Consequently, to achieve good stabilisation, the concentration of TPGS 1000 must be between 2.5% and 15%.

The aqueous solutions proved unstable even in the presence of the antioxidant ascorbic acid (Comparative Example 8) or TPGS 1000 (Comparative Example 9) .

## Claims

1. Non-aqueous injectable formulations comprising lipoic acid or the isomers thereof as active ingredient in concentrations ranging from 2.5% to 25% w/v, at least one non-ionic surfactant in concentrations ranging from 2.5% to 15% w/v, and a polar solvent in concentrations ranging from 50% to 95% w/v.

2. Formulations according to claim 1 wherein the lipoic acid has a concentration ranging from 5% to 15%, preferably 10%.

3. Formulations according to claim 1 or 2 wherein the non-ionic surfactants are selected from polysorbates, polyethoxylated fatty acids, polyethoxylated fatty alcohols, polyethoxylated tocopherols and mixtures thereof.

4. Formulations according to claim 3 wherein the non-ionic surfactants are tocopheryl polyethylene glycol succinates (TPGS).

5. Formulations according to claim 4 wherein the non-ionic surfactant is TPGS 1000, in a concentration ranging from 5 to 15%.

6. Formulations according to any one of claims 1 to 5 wherein the organic solvent is selected from glycerol formal, propylene glycol and a mixture thereof.

7. Formulations according to claim 6 wherein the organic solvent is a glycerol formal/propylene glycol mixture in a weight ratio ranging from 6:4 to 8:2.

8. Formulations according to claim 7 wherein the organic solvent is a glycerol formal/propylene glycol mixture in an 8:2 weight ratio.

9. Formulations according to any one of claims 1 to 8 further comprising an antioxidant.

10. Formulations according to any one of claims 1 to 9 further comprising carnitine in the form of a base, salt or the acylated derivatives thereof.

11. Formulations according to claims 1-10 for use in the prevention and treatment of pathologies caused by oxidative stress and metabolic disorders.

12. Method for stabilising non-aqueous injectable solutions of lipoic acid, which comprises adding 2.5% to 15% w/v of a non-ionic surfactant to a lipoic acid solution at a weight/volume concentration ranging from 2.5 to 25% in a polar solvent.

13. Method according to claim 12 wherein the non-ionic surfactant is a tocopheryl polyethylene glycol succinate.
